**Europäisches Patentamt**

**European Patent Office**

**Office européen des brevets**

(19)

(11) Veröffentlichungsnummer: **0 205 980**
**B1**

(12) # EUROPÄISCHE PATENTSCHRIFT

(45) Veröffentlichungstag der Patentschrift:
**28.12.88**

(21) Anmeldenummer: **86107318.7**

(22) Anmeldetag: **30.05.86**

(51) Int. Cl.⁴: **C 07 D 311/78**

(54) Verfahren zur Herstellung von Perylen-3,4,9,10-tetracarbonsäuredianhydrid.

(30) Priorität: **11.06.85 DE 3520807**

(43) Veröffentlichungstag der Anmeldung:
**30.12.86 Patentblatt 86/52**

(45) Bekanntmachung des Hinweises auf die Patenterteilung:
**28.12.88 Patentblatt 88/52**

(84) Benannte Vertragsstaaten:
**CH DE FR GB IT LI**

(56) Entgegenhaltungen:
**CHEMICAL ABSTRACTS, Band 76, Nr.11, 13. März 1972,
Seite 441, Zusammenfassung 59297z, Columbus, Ohio,
US; Y. NAGAO et al.: "Decomposition of
perylenetetracarboxylic acid diimide in sulfuric acid" &
KOGYO KAGAKU ZASSHI 1971, 74(12), 2500-2**

(73) Patentinhaber: **HOECHST AKTIENGESELLSCHAFT,
Postfach 80 03 20, D-6230 Frankfurt am Main 80 (DE)**

(72) Erfinder: **Spietschka, Ernst, Dr., Am Rödchen 8,
D-6270 Idstein (DE)**
Erfinder: **Urban, Manfred, Steigerwaldstrasse 2a,
D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Perylen-3,4,9,10-tetracarbonsäuredianhydrid in hoher Reinheit und sehr guter Ausbeute auf ökologisch einwandfreie Weise, ausgehend vom Perylen-3,4,9,10-tetracarbonsäurediimid.

Perylen-3,4,9,10-tetracarbonsäuredianhydrid ist ein wichtiges Vorprodukt zur Herstellung von Pigmenten, Küpenfarbstoffen und Fluoreszenzfarbstoffen. An seine Reinheit werden hohe Anforderungen gestellt. Zu seiner Herstellung werden in der Literatur folgende Verfahren beschrieben: Nach der DE-PS 394 794 wird Perylen-3,4,9,10-tetracarbonsäuredianhydrid hergestellt durch Verseifen von Perylen-3,4,9,10-tetracarbonsäurediimid in der 10- 15fachen Menge konz. Schwefelsäure bei etwa 200°C. Nach BIOS Final Report Nr. 1484, Seite 21, wird Perylen-3,4,9,10-tetracarbonsäurediimid in der 10-fachen Menge konzentrischer Schwefelsäure bei Temperaturen von 214 - 216°C verseift und nach der Filtration des rohen Perylen-3,4,9,10-tetracarbonsäuredianhydrids mit der 10fachen Menge konzentrischer Schwefelsäure gewaschen. Das rohe Perylen-3,4,9,10-tetracarbonsäuredianhydrid wird in Kalilauge gelöst und vom nur halbseitig verseiften Perylen-3,4,9,10-tetracarbonsäurediimid (das heisst vom Perylen-3,4,9,10-tetracarbonsäuremonoanhydrid-monoimid) abgetrennt. Bei diesem Verfahren muss mit grossen Mengen konzentrischer Schwefelsäure gearbeitet werden, um gefärbte Nebenprodukte, die durch Oxydations- und Sulfierungsprozesse entstehen, zu entfernen, zumal das Perylen-3,4,9,10-tetracarbonsäurediimid in technischer Qualität mit einer Reinheit < 90% eingesetzt wird. Neben der schlechten Raumzeitausbeute stellen die bei diesen bekannten Verfahren anfallenden Mengen schwefelsaurer Abwässer erhebliche technische Probleme dar. Es bestand deshalb ein Bedarf für ein Verfahren, das ohne Minderung der Ausbeute und der geforderten Reinheit, be i erheblicher Minimierung der Schwefelsäuremenge, insbesondere der Waschschwefelsäuremenge, auf ökologisch einwandfreiem Weg reines Perylen-3,4,9,10-tetracarbonsäuredianhydrid herzustellen erlaubt. Zwar genügt zur Verseifung des Perylen-3,4,9,10-tetracarbonsäurediimids zum entsprechenden Dianhydrid die 2,5- bis 6fache Menge 95%iger Schwefelsäure, bezogen auf technisches Perylen-3,4,9,10-tetracarbonsäurediimid, bei Temperaturen von 210 - 230°C, wobei nach unten die Schwefelsäuremenge durch die Rührbedingungen der Apparatur begrenzt ist. Das hierbei erhaltene rohe Perylen-3,4,9,10-tetracarbonsäuredianhydrid liefert aber auch bei intensiver Wäsche mit der 10fachen Menge konzentrischer Schwefelsäure und Aufarbeitung nach Verfahren des Standes der Technik kein Perylen-3,4,9,10-tetracarbonsäuredianhydrid, das den Anforderungen hinsichtlich Reinheit genügt.

Es wurde nun gefunden, dass man Perylen-3,4,9,10-tetracarbonsäuredianhydrid in hoher Reinheit und sehr guter Ausbeute auf ökologisch einwandfreie Weise herstellen kann, indem man Perylen-3,4,9,10-tetracarbonsäurediimid mit der 2,5 bis 6-fachen Gewichtsmenge 92,5 bis 97,5%iger Schwefelsäure, vorzugsweise 3- bis 5facher Gewichtsmenge 95%iger Schwefelsäure bei Temperaturen von 210 bis 230°C, vorzugsweise 215 bis 220°C, verseift, die hierbei erhaltene Mischung aus Perylen-3,4,9,10-tetracarbonsäuredianhydrid und nur halbseitig verseiftem Perylen-3,4,9,10-tetracarbonsäurediimid (d.h. Perylen-3,4,9,10-tetracarbonsäuremonoanhydrid-monoimid) mit der 0 bis 4-fachen Gewichtsmenge 80 - 95%iger Schwefelsäure, vorzugsweise 80%iger Schwefelsäure, wäscht, anschliessend mit Wasser neutral wäscht, dann die beiden genannten, in Mischung vorliegenden Verbindungen mit Kaliumhydroxid in ihre Kaliumsalze überführt, nach Abtrennung des Dikaliumsalzes des Perylen-3,4,9,10-tetracarbonsäure-monoimids aus der Lösung des Tetrakaliumsalzes der Perylen-3,4,9,10-tetracarbonsäure unter Stickstoff und bei Luft- und Lichtausschluss ein in Wasser suspendiertes oder gelöstes Salz des Eisens, Nickels, Calciums, Magnesiums, Aluminiums, Zinns, Kupfers, Bleis, Zinks oder Mangans zugibt und bei einem pH-Wert > 10 und bei Temperaturen von 0 bis 100°C einwirken lässt, klärt und das Tetrakaliumsalz der Perylen-3,4,9,10-tetracarbonsäure durch Ansäuern in das Perylen-3,4,9,10-tetracarbonsäuredianhydrid überführt und dieses in üblicher Weise, d.h. durch Abfiltrieren, Neutralwaschen mit Wasser und Trocknen, isoliert.

Das so erhaltene Perylen-3,4,9,10-tetracarbonsäuredianhydrid genügt allen Anforderungen hinsichtlich Reinheit und Ausbeute. Die Art der Anionen der Salze der oben angegebenen Metalle richtet sich nach ihrer Verfügbarkeit. Es handelt sich im wesentlichen um Chloride, Sulfate und Carbonate. Die genannten Metallsalze setzt man zweckmässigerweise in einer Menge von 25% bis 200%, vorzugsweise in einer Menge von etwa 50%, bezogen auf das Gewicht des Tetrakaliumsalzes der Perylen-3,4,9,10-tetracarbonsäure, zu. Beim Zusatz von etwa 50%, gegebenenfalls unter Berücksichtigung des Kristallwassers und des Molekulargewichts, erzielt man die besten Reinigungseffekte. Es ist weiterhin von Vorteil, Aktivkohle zuzusetzen. Es war überraschend, dass bei dieser Arbeitsweise nur die Verunreinigungen in Form ihrer Salze abgeschieden werden und nicht die schwerlöslichen Salze des Perylen-3,4,9,10-tetracarbonsäuredianhydrids mit den Kationen der genannten Metalle. Der bei dieser Klärung anfallende Klärschlamm kann ferner dem schwefelsauren Filtrat des rohen Perylen-3,4,9,10-tetracarbonsäuredianhydrids nach Verdünnen auf < 20% zugesetzt und der ausfallende Rückstand abgetrennt werden. Durch diese Massnahme erfolgt nicht nur Entfärbung des rot-gefärbten Abwassers, sondern gleichzeitig werden für den biologischen Abbau störende toxische Verbindungen entfernt. Selbstverständlich ist der Reinigungseffekt und die Abwasserverbesserung auch erreichbar, wenn in grösserer schwefelsaurer Verdünnung gearbeitet wird.

Durch die nachstehenden Beispiele soll das erfindungsgemässe Verfahren erläutert werden, ohne es darauf zu beschränken.

*Beispiel 1*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält die Temperatur 2 Stunden aufrecht. Anschliessend lässt man auf Raumtemperatur abkühlen, saugt den gebildeten Niederschlag ab und wäscht diesen mit Wasser neutral. Nach Trocknen bei 80°C erhält man 174,3 g rohes Perylen-3,4,9,10-tetracarbonsäuredianhydrid. Diese werden unter Lichtausschluss in eine Lösung aus 6 l Wasser und 140 g Kaliumhydroxid (85%ig) eingetragen. Unter Überleiten von Stickstoff wird die Lösung auf 80 bis 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 38,8 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 60 g Eisen(II)-sulfat und 600 ml Wasser zutropfen, rührt 1 Stunde bei 25°C nach, klärt und lässt dann das Filtrat auf 600 g konzentrierte Salzsäure tropfen, hält 1 Stunde bei 80-90°C, saugt den Niederschlag ab, wäscht diesen mit Wasser neutral und trocknet ihn bei 80°C.

Man erhält 120,9 g Perylen-3,4,9,10-tetracarbonsäuredianhydrid.

Die Reinheit des Perylen-3,4,9,10-tetracarbonsäuredianhydrids wird nach folgender Methode bestimmt:

a) *Lösen der Probe:*

2,5 g Perylen-3,4,9,10-tetracarbonsäuredianhydrid werden in 40 ml Wasser eingetragen. Nach Zugeben von 1,75 g Kaliumyhydroxid 85%ig wird unter Stickstoff und bei Luftausschluss 30 Minuten bei 50°C gerührt, wobei Lösung erfolgt. Dann wird über ein Faltenfilter in ein mit Stickstoff gespültes Gefäss filtriert. Die erhaltene Lösung wird im Dunkeln aufbewahrt bis zur Messung.

b) *Messung:*

Gemessen wird mit dem Spektralphotometer DU 7 (der Firma Beckmann) unter Verwendung einer 1 cm Küvette. Registriert wird die Basislinie des Lösungsgemisches ohne Probeeinwaage. Die Aufnahme der Absorptionsspektren erfolgt zwischen 800 nm und 500 nm.

c) *Ergebnis:*

Die gegen die Extinktion bei 780 nm gemessenen Extinktionswerte bei 580 nm und 570 nm werden abgelesen. Für das gemäss Beispiel 1 erhaltene Perylen-3,4,9,10-tetracarbonsäuredianhydrid ergeben sich folgende Extinktionswerte:

1,43 bei 580 nm und
2,50 bei 570 nm.

Setzt man dem mit Wasser verdünnten Schwefelsäurefiltrat den Klärrückstand aus Tierkohle und Eisen(II)-sulfat zu, so erhält man nach der Klärung ein nicht fischtoxisches, nicht bakterientoxisches, biologisch hervorragend abbaubares Abwasser mit einer Abbaurate von 81,4%. Ohne diese Massnahme liegt die Abbaurate bei nur 8%, wobei überdies die Bakterientoxität erhalten bleibt.

*Beispiel 2*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 171,6 g rohes Perylen-3,4,9,10-tetracarbonsäuredianhydrid. Diese werden unter Lichtausschluss in eine Lösung aus 6 l Wasser und 140 g Kaliumhydroxid (85%ig) eingetragen. Unter Überleiten von Stickstoff wird die Lösung auf 80 bis 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 34,1 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 60 g Eisen(II)-sulfat und 600 ml Wasser zutropfen, rührt 1 Stunde bei 25°C nach, klärt und lässt dann das Filtrat auf 600 g konzentrische Salzsäure tropfen, hält 1 Stunde bei 80-90°C, saugt den Niederschlag ab, wäscht diesen mit Wasser neutral und trocknet ihn bei 80°C.

Man erhält 125,1 g Perylen-3,4,9,10-tetracarbonsäuredianhydrid, welchem die Extinktionswerte

0,76 bei 580 nm und
1,39 bei 570 nm

zukommen.

(Die Bestimmung der Reinheit erfolgt nach der in Beispiel 1 angegebenen Methode).

*Beispiel 3*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 170,9 g rohes Perylen-3,4,9,10-tetracarbonsäuredianhydrid.

Diese werden unter Lichtausschluss in eine Lösung aus 6 l Wasser und 140 g Kaliumhydroxid (85%ig) eingetragen. Unter Überleiten von Stickstoff wird die Lösung auf 80 - 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 35,6 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 120 g Kaliumhydroxid (85%ig) ein und leitet dann Stickstoff über. Nach 1stündigem Nachrühren bei 25°C lässt man eine Lösung von 120 g Eisen(II)-sulfat und 600 ml Wasser zutropfen, rührt 1 Stunde bei 25°C nach, klärt und lässt dann das Filtrat auf 600 g konzentrierte Salzsäure tropfen, hält 1 Stunde bei 80 - 90°C, saugt den Niederschlag ab, wäscht diesen mit Wasser neutral und trocknet ihn bei 80°C.

Man erhält 130,8 g Perylen-3,4,9,10-tetracarbonsäuredianhydrid, welchem die Extinktionswerte

    0,84 bei 580 nm und
    1,46 bei 570 nm

zukommen.

(Die Bestimmung der Reinheit erfolgt nach der in Beispiel 1 angegebenen Methode.)

*Beispiel 4*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 170,9 rohes Perylen-3,4,9,10-tetracarbonsäuredianhydrid.

Diese werden unter Lichtausschluss in eine Lösung aus 6 l Wasser und 140 g Kaliumhydroxid (85%ig) eingetragen. Unter Überleiten von Stickstoff wird die Lösung auf 80 - 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 35,6 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 110 g Nickel(II)-sulfat krist. in 600 ml Wasser zutropfen, rührt 1 Stunde bei 25°C nach, klärt und lässt dann das Filtrat auf 600 g konzentrierte Salzsäure tropfen, hält 1 Stunde bei 80 - 90°C, saugt den Niederschlag ab, wäscht diesen mit Wasser neutral und trocknet ihn bei 80°C.

Man erhält 121,8 g Perylen-3,4,9,10-tetracarbonsäuredianhydrid, welchem die Extinktionswerte

    0,80 bei 580 nm und
    1,42 bei 570 nm

zukommen.

(Die Bestimmung der Reinheit erfolgt nach der in Beispiel 1 angegebenen Methode.)

*Beispiel 5*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 172,1 rohes Perylen-3,4,9,10-tetracarbonsäuredianhydrid.

Diese werden unter Lichtausschluss in eine Lösung aus 6 l Wasser und 140 g Kaliumhydroxid (85%ig) eingetragen. Unter Überleiten von Stickstoff wird die Lösung auf 80 - 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 33,3 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 263 g Aluminiumsulfat krist. in 600 ml Wasser zutropfen, rührt 1 Stunde bei 25°C nach, klärt und lässt dann das Filtrat auf 600 g konzentrierte Salzsäure tropfen, hält 1 Stunde bei 80 - 90°C, saugt den Niederschlag ab, wäscht diesen mit Wasser neutral und trocknet ihn bei 80°C.

Man erhält 119,6 g Perylen-3,4,9,10-tetracarbonsäuredianhydrid, welchem die Extinktionswerte

    0,69 bei 580 nm und
    1,29 bei 570 nm

zukommen.

(Die Bestimmung der Reinheit erfolgt nach der in Beispiel 1 angegebenen Methode.)

*Beispiel 6*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 170,9 rohes Perylen-3,4,9,10-tetracarbonsäuredianhydrid.

Diese werden unter Lichtausschluss in eine Lösung aus 6 l Wasser und 140 g Kaliumhydroxid (85%ig) eingetragen. Unter Überleiten von Stickstoff wird die Lösung auf 80 - 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 36,1 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 84 g Magnesiumchlorid krist. und 600 ml Wasser zutropfen, rührt 1 Stunde bei 25°C nach, klärt und lässt dann das Filtrat auf 600 g konzentrierte Salzsäure tropfen, hält 1 Stunde bei 80 - 90°C, saugt den Niederschlag ab, wäscht diesen mit Wasser neutral und trocknet ihn bei 80°C.

Man erhält 126,1 g Perylen-3,4,9,10-tetracarbonsäuredianhydrid, welchem die Extinktionswerte

0,82 bei 580 nm und
1,44 bei 570 nm

zukommen.

(Die Bestimmung der Reinheit erfolgt nach der in Beispiel 1 angegebenen Methode.)

*Beispiel 7*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 172,3 rohes Perylen-3,4,9,10-tetracarbonsäuredianhydrid.

Diese werden unter Lichtausschluss in eine Lösung aus 6 l Wasser und 140 g Kaliumhydroxid (85%ig) eingetragen. Unter Überleiten von Stickstoff wird die Lösung auf 80 - 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 34,3 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 111,6 g Calciumchlorid krist. und 600 ml Wasser zutropfen, rührt 1 Stunde bei 25°C nach, klärt und lässt dann das Filtrat auf 600 g konzentrierte Salzsäure tropfen, hält 1 Stunde bei 80 - 90°C, saugt den Niederschlag ab, wäscht diesen mit Wasser neutral und trocknet ihn bei 80°C.

Man erhält 121,2 g Perylen-3,4,9,10-tetracarbonsäuredianhydrid, welchem die Extinktionswerte

0,65 bei 580 nm und
1,26 bei 570 nm

zukommen.

(Die Bestimmung der Reinheit erfolgt nach der in Beispiel 1 angegebenen Methode.)

*Beispiel 8*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 172,4 rohes Perylen-3,4,9,10-tetracarbonsäuredianhydrid.

Diese werden unter Lichtausschluss in eine Lösung aus 6 l Wasser und 140 g Kaliumhydroxid (85%ig) eingetragen. Unter Überleiten von Stickstoff wird die Lösung auf 80 - 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 35,0 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 60 g Eisen(III)-chlorid und 600 ml Wasser zutropfen, rührt 1 Stunde bei 25°C nach, klärt und lässt dann das Filtrat auf 600 g konzentrierte Salzsäure tropfen, hält 1 Stunde bei 80 - 90°C, saugt den Niederschlag ab, wäscht diesen mit Wasser neutral und trocknet ihn bei 80°C.

Man erhält 125,6 g Perylen-3,4,9,10-tetracarbonsäuredianhydrid, welchem die Extinktionswerte

0,77 bei 580 nm und
1,43 bei 570 nm

zukommen.

(Die Bestimmung der Reinheit erfolgt nach der in Beispiel 1 angegebenen Methode.)

*Beispiel 9*

In 800 g vorgelegte Schwefelsäure (95%ig) werden bei Raumtemperatur 200 g Perylen-3,4,9,10-tetracarbonsäurediimid (89,4%ig) eingetragen. Dann erhöht man die Temperatur der Mischung auf 220°C und hält diese Temperatur 2 Stunden aufrecht. Anschliessend lässt man auf Raumtemperatur abkühlen, saugt den gebildeten Niederschlag ab, wäscht diesen zunächst mit 280 g 80%iger Schwefelsäure, dann mit Wasser neutral. Nach Trocknen bei 80°C erhält man 174,3 rohes Perylen-3,4,9,10-tetracarbonsäuredianhydrid.

Diese werden unter Lichtausschluss in eine Lö-sung aus 6 l Wasser und 140 g Kaliumhydroxid (85%ig) eingetragen. Unter Überleiten von Stickstoff wird die Lösung auf 80 - 90°C erwärmt und 90 Minuten bei dieser Temperatur nachgerührt. Anschliessend lässt man auf 25°C abkühlen, stellt den pH-Wert auf 8 - 9 ein, saugt vom festen Rückstand ab und wäscht diesen mit 100 ml Wasser. (Der Rückstand stellt 38,7 g des Dikaliumsalzes des nur halbseitig verseiften Perylen-3,4,9,10-tetracarbonsäurediimids, d.h. das Dikaliumsalz des Perylen-3,4,9,10-tetracarbonsäuremonoimids dar, welches zweckmässigerweise dem nächsten Ansatz zugesetzt wird.) In das Filtrat trägt man unter Lichtausschluss 60 g Kaliumhydroxid (85%ig) und 60 g Tierkohle ein und leitet dann Stickstoff über. Nach 1stündigem Nachrühren bei 25°C lässt man eine Lösung von 48,8 g Kupfercarbonat und 600 ml Wasser zutropfen, rührt 1 Stunde bei 25°C nach, klärt und lässt dann das Filtrat auf 600 g konzentrierte Salzsäure tropfen, hält 1 Stunde bei 80 - 90°C, saugt den Niederschlag ab, wäscht diesen mit Wasser neutral und trocknet ihn bei 80°C.

Man erhält 133,7 g Perylen-3,4,9,10-tetracarbonsäuredianhydrid, welchem die Extinktionswerte

0,99 bei 580 nm und
1,54 bei 570 nm

zukommen.
(Die Bestimmung der Reinheit erfolgt nach der in Beispiel 1 angegebenen Methode.)


**Patentansprüche**

1. Verfahren zur Herstellung von Perylen-3,4,9,10-tetracarbonsäuredianhydrid in hoher Reinheit und sehr guter Ausbeute auf ökologisch einwandfreie Weise, dadurch gekennzeichnet, dass man Perylen-3,4,9,10-tetracarbonsäurediimid mit der 2,5 bis 6fachen Gewichtsmenge 92,5 bis 97,5%iger Schwefelsäure bei Temperaturen von 210 bis 230°C verseift, die erhaltene Mischung aus Perylen-3,4,9,10-tetracarbonsäuredianhydrid und Perylen-3,4,9,10-tetracarbonsäure-monoanhydrid-monoimid mit der 0 bis 4fachen Gewichtsmenge 80 95%iger Schwefelsäure wäscht, anschliessend mit Wasser neutral wäscht, die beiden genannten, in Mi-schung vorliegenden Verbindungen mit Kaliumhy-droxid in ihre Kaliumsalze überführt, nach Abtrennung des Dikaliumsalzes des Perylen-3,4,9,10-tetracarbonsäure-monoimids aus der Lösung des Tetrakaliumsalzes der Perylen-3,4,9,10-tetracarbonsäure unter Stickstoff und bei Luft- und Lichtausschluss ein in Wasser suspendiertes oder gelöstes Salz des Eisens, Nickels, Calciums, Magnesiums, Aluminiums, Zinns, Kupfers, Bleis, Zinks oder Mangans zugibt und bei einem pH-Wert > 10 und bei Temperaturen von 0 bis 100°C einwirken lässt, anschliessend klärt und das Tetrakaliumsalz der Perylen-3,4,9,10-tetracarbonsäure durch Ansäuern in das Perylen-3,4,9,10-tetracarbonsäuredianhydrid überführt und dieses in üblicher Weise isoliert.

2. Verfahren nach Anspruch 1, dadurch gekennzeichnet, dass man wässrige Lösungen oder Suspensionen der Chloride, Sulfate oder Carbonate des Eisens, Nickels, Calciums, Magnesiums, Aluminiums, Zinns, Kupfers, Bleis, Zinks oder Mangans zusetzt.


**Claims**

1. A process for the preparation of perylene-3,4,9,10-tetracarboxylic dianhydride of high purity and in a very good yield and in an ecologically unobjectionable manner, which comprises saponifying perylene-3,4,9,10-tetracarboxylic acid diimide with a 2.5-fold to 6-fold amount by weight of 92.5 to 97.5% sulfuric acid at temperatures of 210 to 230°C, washing the resulting mixture of perylene-3,4,9,10-tetracarboxylic dianhydride and perylene-3,4,9,10-tetracarboxylic acid monoanhydride-monoimide with a 0-fold to 4-fold amount by weight of 80-95% sulfuric acid, and then washing it with water until it is neutral, converting the two said compounds present in the mixture into their potassium salts by means of potassium hydroxide, adding, after removing the dipotassium salt of perylene-3,4,9,10-tetracarboxylic acid monoimide from the solution of the tetrapotassium salt of perylene-3,4,9,10-tetracarboxylic acid, under nitrogen and with the exclusion of air and light, a salt of iron, nickel, calcium, magnesium, aluminium, tin, copper, lead, zinc or manganese, suspended or dissolved in water, and allowing it to act at a pH > 10 and at temperatures from 0 to 100°C, and then clarifying the mixture and converting the tetrapotassium salt of perylene-3,4,9,10-tetracarboxylic acid by acidification into perylene-3,4,9,10-tetracarboxylic dianhydride and isolating the latter in a customary manner.

2. The process as claimed in claim 1, wherein aqueous solutions or suspensions of the chlorides, sulfates or carbonates of iron, nickel, calcium, magnesium, aluminium, tin, copper, lead, zinc or manganese are added.


**Revendications**

1. Procédé pour préparer le dianhydride de l'acide pérylène-tétracarboxylique-3,4,9,10 à l'état très pur et avec un très bon rendement, d'une manière donnant toute satisfaction du point de vue écologique, procédé caractérisé en ce qu'on saponifie le diimide de l'acide pérylène-tétracarboxylique-3,4,9,10, à des températures de 210 à 230°C, avec une quantité en poids d'acide sulfurique à 92,5 - 97,5% représentant de 2,5 à 6 fois son poids, on lave le mélange obtenu, qui est constitué de dianhydride de l'acide pérylène-tétracarboxylique-3,4,9,10 et de mono-imide mono-anhydride de l'acide pérylène-tétracarboxylique-3,4,9,10, avec une quantité en poids d'acide sulfurique à 80 - 95% représentant de 0 à 4 fois son poids, puis on le lave à l'eau jusqu'à neutralité, on transforme les deux composés mélangés cités, au moyen d'hydroxyde de potassium, en leurs sels potassiques, puis, après avoir séparé le sel dipotassique du mono-imide de l'acide pérylène-tétracarboxylique-3,4,9,10 de la solution du sel tétrapotassique de l'acide pérylène-tétracarboxylique-3,4,9,10, on ajoute, tout en opérant sous azote et à l'abri de l'air et de la lumière, un sel de fer, de nickel, de calcium, de magnésium, d'aluminium, d'étain, de cuivre, de plomb, de zinc ou de manganèse en suspension ou dissous dans de l'eau et on laisse agir à un pH supérieur à 10 et à des températures de 0 à 100°C, après quoi on clarifie, on transforme le sel tétrapotassique de l'acide pérylène-tétracarboxylique-3,4,9,10, par acidification, en le dianhydride de l'acide pérylène-tétracarboxylique-3,4,9,10, et on isole celui-ci de la manière habituelle.

2. Procédé selon la revendication 1, caractérisé en ce qu'on ajoute des solutions ou suspensions aqueuses des chlorures, sulfates ou carbonates de fer, de nickel, de calcium, de magnésium, d'aluminium, d'étain, de cuivre, de plomb, de zinc ou de manganèse.